# EUROPÄISCHE PATENTANMELDUNG

(11) **EP 2 028 203 A1**
(43) Veröffentlichungstag der Anmeldung: **25.02.2009**
(21) Anmeldenummer: 08160178.3
(22) Anmeldetag: 11.07.2008
(51) Int. Cl.: C08G 18/02, C08G 18/79, C09D 127/06

(54) **Phthalatfreie Isocyanuratzubereitungen**

(30) Priorität: 26.07.2007 DE 102007034977
(71) Anmelder: LANXESS Deutschland GmbH, 51369 Leverkusen (DE)
(72) Erfinder: Hansel, Jan-Gerd, 51469, Bergisch Gladbach (DE); Augustin, Thomas, 51065, Köln (DE)

(57) **Zusammenfassung**

Die vorliegende Erfindung betrifft neue monomerenarme, niedrigviskose Zubereitungen aus isocyanatgruppenhaltigen Isocyanuraten und phthalatfreien Weichmachern, ihre Verwendung als Haftvermittler für Beschichtungsmittel auf Basis von weichgemachtem Polyvinylchlorid, sowie Beschichtungen und beschichtete Substrate.

## Beschreibung

Die vorliegende Erfindung betrifft neue monomerenarme, niedrigviskose Zubereitungen aus isocyanatgruppenhaltigen Isocyanuraten und phthalatfreien Weichmachern, ihre Verwendung als Haftvermittler für Beschichtungsmittel auf Basis von weichgemachtem Polyvinylchlorid (PVC), sowie Beschichtungen und beschichtete Substrate.

Es ist bekannt, die Haftfähigkeit von weichgemachtem PVC auf Substraten dadurch zu verbessern, dass dem weichgemachten PVC ein isocyanatgruppenhaltiger Haftvermittler zugesetzt wird. Eine derart verbesserte Haftfähigkeit ist beispielsweise wichtig, wenn mit einem PVC-Belag versehene synthetische Textilstoffe hergestellt werden sollen. Als Haftvermittler werden bevorzugt isocyanatgruppenhaltige Isocyanurate eingesetzt, die sich durch Oligomerisierung, insbesondere Trimerisierung, aus Diisocyanaten herstellen lassen. Die hierfür am besten geeigneten Diisocyanate sind die kommerziell gut verfügbaren Mischungen der isomeren Diisocyanatotoluole (TDI), bestehend hauptsächlich aus 2,4-Diisocyanatotoluol (2,4-TDI) und 2,6-Diisocyanatotoluol (2,6-TDI). Diese lassen sich auf einfache Weise nahezu vollständig in isocyanatgruppenhaltige Isocyanurate umsetzen. Der nahezu vollständige Umsatz ist notwendig, denn aus Gründen der Arbeits- und Produktsicherheit ist erforderlich, dass der Restgehalt an Diisocyanaten in der Haftvermittler-Zubereitung unter 1,0 Gew.-% bleibt. Weniger geeignet sind die ebenfalls gut verfügbaren Diisocyanatodiphenylmethane (MDI), die sich im Vergleich mit TDI schlechter trimerisieren lassen und so zu einem unerwünscht hohen Restgehalt an Diisocyanaten führen können. Darüber hinaus zeigen isocyanatgruppenhaltige Isocyanurate auf Basis von MDI eine schlechte Löslichkeit und neigen zur Kristallisation.

Isocyanatgruppenhaltige Isocyanurate lassen sich als Haftvermittler vor allem dann einfach handhaben, wenn sie in Form einer Lösung in einem Weichmacher angewendet werden. Die Herstellung der isocyanatgruppenhaltigen Isocyanurate aus TDI wird praktischer Weise ebenfalls in dem als Lösungsmittel verwendeten Weichmacher durchgeführt. Derartige Haftvermittler und weichmacherhaltige Haftvermittler-Zubereitungen, ihre Herstellung und ihre Anwendung sind beispielsweise in DE 24 19 016 A1 beschrieben.

Weichmacher sind Stoffe, die beim Mischen mit dem an sich harten und spröden PVC einen weichen, zähen Werkstoff ergeben, das so genannte weichgemachte PVC. Bekannte Weichmacher sind beispielweise die Ester der Phthalsäure, Adipinsäure oder Benzoesäure. In weichgemachtem PVC können diese Weichmacher in großen Mengen enthalten sein, teilweise über 50 Gew.-% des weichgemachten PVC. Der Weichmacher kann sich unter Gebrauchsbedingungen oberflächlich absondern oder in benachbarte Materialien übergehen. Bei der Verwendung von weichgemachtem PVC besteht daher die Gefahr einer Kontamination von Mensch und Umwelt mit dem Weichmacher. Vor dem Hintergrund dieser Problematik wird in letzter Zeit vermehrt von den verwendeten Weichmachern gefordert, dass sie für den Menschen harmlos und nicht bioakkumulierend sind.

Nach der in der Europäischen Union gültigen Richtlinie 2005/84/EG dürfen beispielsweise die Weichmacher Di(2-ethylhexyl)phthalat, Dibutylphthalat und Benzylbutylphthalat nicht mehr in Spielzeug oder Babyartikeln verwendet werden und die Weichmacher Diisononylphthalat, Diisodecylphthalat und Di-n-octylphthalat nicht mehr in Spielzeug oder Babyartikeln, die von Kindern in den Mund genommen können, verwendet werden. Angesichts dieser Einschränkungen, die für manche Konsumenten als unübersichtlich und verunsichernd erscheinen können, gehen viele Hersteller dazu über, bei der Herstellung von weichgemachtem PVC generell auf alle phthalathaltigen Weichmacher zu verzichten. Daher besteht ein Bedarf an phthalatfreien Weichmachern, die hinsichtlich Verarbeitbarkeit und Nutzeigenschaften das Leistungsniveau von phthalathaltigen Weichmachern erreichen.

Als phthalatfreie Weichmacher werden im Zusammenhang mit der vorliegenden Erfindung solche Weichmacher bezeichnet, die keine Phthalsäuredialkylester enthalten, insbesondere Weichmacher, die weniger als 0,1 Gew.-% Phthalsäuredialkylester enthalten.

Der Verzicht auf phthalathaltige Weichmacher wird nun auch von weichmacherhaltigen Haftvermittler-Zubereitungen gefordert, insbesondere für sensible Anwendungen wie Spielzeug oder Babyartikel. Es besteht daher ein großer Bedarf an Haftvermittler-Zubereitungen, die keine Phthalate enthalten, aber dennoch die guten Haftungseigenschaften phthalathaltiger Haftvermittler-Zubereitungen aus dem Stand der Technik aufweisen. Weiterhin wird gefordert, dass die Zubereitungen klar und frei von Feststoffen sind, keine flüchtigen Lösungsmittel enthalten und für eine gute Verarbeitbarkeit eine Viskosität bei 23 °C von kleiner als 30.000 mPas aufweisen. Der Restgehalt an Diisocyanaten soll weniger als 1,0 Gew.-% betragen. Eine Kombination aller dieser Produkteigenschaften ist im Stand der Technik bisher nicht beschrieben.

So sind die in WO 2005 70984 A1 beschriebenen Haftvermittler-Zubereitungen auf Basis von Diisononylphthalat für sensible Anwendungen nicht mehr geeignet. In DE 25 51 634 A1 und EP 1 378 529 A1 wird behauptet, als Haftvermittler geeignete isocyanatgruppenhaltige Isocyanurate auf Basis von TDI lassen sich in beliebigen Lösungsmitteln, darunter auch in phthalatfreien Weichmachem, herstellen. Die unten angeführten Vergleichsbeispiele zeigen jedoch, dass keineswegs alle phthalatfreien Weichmacher zu Haftvermittler-Zubereitungen führen, die die beschriebenen Anforderungen erfüllen. DE 30 41 732 A1 beschreibt als Haftvermittler geeignete Lösungen von isocyanatgruppenhaltigen Isocyanuraten, die allerdings aus MDI hergestellt werden. Diese Lösungen sind aus den oben genannten Gründen ungeeignet.

Aufgabe der vorliegenden Erfindung war es daher, als Haftvermittler geeignete Zubereitungen von isocyanatgruppenhaltigen Isocyanuraten zur Verfügung zu stellen, die zwar phthalatfreie Weichmacher enthalten, aber in ihren mechanischen Eigenschaften, wie z. B. Haftfestigkeiten, das Niveau der phthalathaltigen Haftvermittler-Zubereitungen aus WO 2005 70984 A1 erreichen. Die isocyanatgruppenhaltigen Isocyanurate sollen auf großtechnisch verfügbaren Isomerenmischungen des TDI basieren, insbesondere soll die Verwendung von reinem 2,4-TDI aus wirtschaftlichen Gründen vermieden werden. Die Zubereitungen sollen klar sein, ihre Viskosität soll < 30.000 mPas bei 23 °C und der Gehalt an freiem TDI (alle Isomere) soll < 1,0 Gew.-% betragen.

Diese Aufgabe wird gelöst durch Zubereitungen von isocyanatgruppenhaltigen Isocyanuraten mit einer Viskosität von < 30.000 mPas bei 23 °C und einem Anteil an freiem TDI von ≤ 1,0 Gew.-% (Summe aller TDI-Isomere), dadurch gekennzeichnet, dass sie
A) 15 bis 50 Gew.-% isocyanatgruppenhaltige Isocyanurate, die aus einer Mischung isomerer Diisocyanatotoluole enthaltend 65 bis 95 Gew.-% 2,4-Diisocyanatotoluol und 5 bis 35 Gew.-% 2,6-Diisocyanatotoluol unter Katalyse durch Dialkylaminogruppen enthaltende phenolische Katalysatoren und in Abwesenheit aliphatischer Hydroxyl- und/oder Urethangruppen hergestellt wurden, und
B) 85 bis 50 Gew.-% Alkansulfonsäurearylester-haltige, phthalatfreie Weichmacher enthalten.

In einer bevorzugten Ausführungsform der Erfindung enthalten die Zubereitungen 20 bis 35 Gew.-% isocyanatgruppenhaltige Isocyanurate und 80 bis 65 Gew.-% Alkansulfonsäurearylester-haltige, phthalatfreie Weichmacher.

Zur Herstellung der Komponente A) werden großtechnisch verfügbare Gemische bestehend im Wesentlichen aus 2,4-TDI und 2,6-TDI verwendet. Bevorzugt enthalten die TDI-Isomerenmischungen 75 bis 85 Gew.-% 2,4-TDI im Gemisch mit 15 bis 25 Gew.-% 2,6-TDI. Ein Beispiel für diese bevorzugt zu verwendenden TDI-Isomerenmischungen ist das kommerziell von der Bayer Material Science AG erhältliche Produkt Desmodur® T80.

Zur Herstellung der Komponente A) werden weiterhin Dialkylaminogruppen enthaltende phenolische Katalysatoren verwendet, die dem Fachmann unter der Bezeichnung Mannich-Basen hinlänglich bekannt sind. Die Synthese geeigneter Mannich-Basen ist beispielsweise in DE 25 51 634 A1 und WO 2005 70984 A1 beschrieben.

Die Herstellung der Komponente A) geschieht durch Trimerisierung der Diisocyanatmischungen nach an sich bekannten Verfahren, wie sie beispielsweise in WO 2005 70984 A1 beschrieben sind. Vorteilhaft wird die Trimerisierung in Gegenwart der Weichmacherkomponente B) durchgeführt. Die Trimerisierungsreaktion erfolgt im Temperaturbereich von 40 bis 140 °C, vorzugsweise 40 bis 80 °C. Wenn der Gehalt an freiem TDI in der Reaktionsmischung unter 1,0 Gew.-% liegt, wird die Trimerisierung durch thermische Zersetzung des Katalysators oder aber bevorzugt durch Zugabe eines Katalysatorengifts abgebrochen. Das Produkt enthält dann 3 bis 7 Gew.-% Isocyanatgruppen.

Für den Abbruch der Trimerisierungsreaktion geeignete Katalysatorengifte sind Säuren oder Säurederiate, wie beispielsweise Perfluorbutansulfonsäure, Propionsäure, die isomeren Phthalsäurechloride, Benzoesäure oder Benzoylchlorid, sowie Quarternierungsmittel, wie beispielsweise para-Toluolsulfonsäuremethylester, Diethylsulfat, Phosphorsäuremono- oder diester. Bevorzugt wird als Katalysatorgift para-Toluolsulfonsäuremethylester eingesetzt.

Die Alkansulfonsäurearylester-haltigen, phthalatfreien Weichmacher der Komponente B) enthalten erfindungsgemäß < 0,1 Gew.-% Phthalsäuredialkylester und > 0,1 % Alkansulfonsäurearylester. In einer bevorzugten Ausführungsform der Erfindung werden als Komponente B) phthalatfreie Weichmacher mit > 90 Gew.-% Alkansulfonsäurearylester eingesetzt. Die Alkansulfonsäurearylester leiten sich von primären oder sekundären C₆- bis C₂₀-Alkansulfonsäuren oder Mischungen dieser Sulfonsäuren ab. Bei den in den Alkansulfonsäurearylester enthaltenen Arylresten handelt es sich um Phenyl, mit ein bis fünf C₁- bis C₂₀-Alkylresten substituiertes Phenyl oder mit einem C₆- bis C₁₀-Arylrest substituiertes Phenyl. Besonders bevorzugt ist als Komponente B) eine Mischung von Alkansulfonsäurephenylestern enthalten. Solche Mischungen sind im industriellen Maßstab gut verfügbar, wie beispielsweise die Produkte Mesamoll® oder Mesamoll® II der Lanxess Deutschland GmbH.

Die erfindungsgemäßen Zubereitungen sind klare, leicht gelbliche Flüssigkeiten, die auch nach mehrwöchiger Lagerung weder zur Kristallisation noch zur Bildung von Ausfällungen oder Phasentrennung neigen. Außerdem zeichnen sie sich auch nach Lagerung durch einen äußerst geringen Gehalt an freiem TDI aus, was wegen des relativ niedrigen Siedepunktes dieses toxikologisch bedenklichen Diisocyanats ein besonderer Vorteil der erfindungsgemäßen Zubereitungen ist.

Da als Haftvermittler geeignete Zubereitungen isocyanatgruppenhaltiger Isocyanurate nach dem Stand der Technik am besten durch Trimerisation von Diisocyanaten im Weichmacher hergestellt werden und der Verlauf der Trimerisierungsreaktion nicht nur vom Katalysator, sondern auch beispielsweise durch den eingesetzten Weichmacher, die Isomerenzusammensetzung des TDI oder gleichzeitig anwesende beispielsweise hydroxylgruppenhaltige Verbindungen beeinflusst wird, war es nicht zu erwarten, dass gerade die erfindungswesentliche Kombination von Weichmacher, Katalysator und Höchstmenge an 2,6-TDI bei gleichzeitiger Abwesenheit von Hydroxyverbindungen Haftvermittler-Zubereitungen mit den geforderten Eigenschaften liefern würde. Tatsächlich zeigen die unten angeführten Vergleichsbeispiele 2 und 3, dass die dieser Erfindung zugrunde liegende Aufgabe nicht mit beliebigen phthalatfreien Weichmachern zu lösen ist.

Die erfindungsgemäßen Zubereitungen eignen sich als Haftvermittler für weichgemachtes PVC und insbesondere als haftvermittelnde Zusätze für PVC-Plastisole. Besonders vorteilhaft werden die erfindungsgemäßen Zubereitungen als Haftvermittler zwischen Substraten aus Chemiefasern mit gegenüber Isocyanatgruppen reaktionsfähigen Gruppen, wie z. B. Polyamid- oder Polyesterfasern, und PVC-Plastisolen bzw. Weich-PVC-Schmelzen verwendet. Selbstverständlich kann mit den erfindungsgemäßen Lösungen auch die Haftung von weichgemachtem PVC bzw. PVC-Plastisolen an flächigen Substraten, wie beispielsweise an Folien, verbessert werden.

Daher ist ein weiterer Gegenstand der vorliegenden Erfindung die Verwendung der erfindungsgemäßen Zubereitungen als Haftvermittler für Beschichtungsmittel auf Basis von weichgemachtem PVC.

Bei der erfindungsgemäßen Verwendung der erfindungsgemäßen Zubereitungen kann beispielsweise so vorgegangen werden, dass die erfindungsgemäßen Zubereitungen auf die zu beschichtenden Substrate gedruckt, gerakelt, gerastert oder gesprüht oder durch Tauchen aufgebracht werden. Je nach herzustellendem Artikel werden auf die so vorbehandelten Substratoberflächen eine oder mehrere haftvermittlerfreie PVC-Schichten, z. B. als Plastisole oder durch Extrusions- oder Schmelzwalzenbeschichtung oder durch Laminierung, aufgebracht. Besonders bevorzugt können die erfindungsgemäßen Zubereitungen auch einem PVC-Plastisol vor dessen Applikation zugesetzt werden.

Die erfindungsgemäßen Zubereitungen werden normalerweise in solchen Mengen eingesetzt, dass, bezogen auf weichmacherfreies PVC der Beschichtungsmasse, 0,5 bis 200 Gew.-%, vorzugsweise 2 bis 30 Gew.-% isocyanatgruppenhaltige Isocyanurate vorliegen. Die erfindungsgemäßen Lösungen können jedoch auch in beliebigen anderen, dem jeweiligen Anwendungsgebiet angepassten Mengen eingesetzt werden.

Die Herstellung der fertigen Schichten, also die Reaktion der Isocyanatgruppen des Haftvermittlers mit dem Substrat und die Gelierung der PVC-Schicht, erfolgt unabhängig von der Art des Auftrags nach der üblichen Weise bei höheren Temperaturen, wobei je nach Zusammensetzung der PVC-Schichten Temperaturen zwischen 110 und 210 °C angewandt werden.

Ein weiterer Gegenstand der vorliegenden Erfindung sind Beschichtungen und beschichtete Substrate für Textilien oder Gewebe, die unter Verwendung der oben beschriebenen Haftvermittler-Zubereitungen erhältlich sind. Die erfindungsgemäßen Zubereitungen eignen sich als Haftvermittler für Beschichtungen auf Basis von weichgemachtem PVC, insbesondere zur Herstellung von Planen, Billboards, Traglufthallen und anderen textilen Bauten, flexiblen Behältern, Zeltdächern, Markisen, Schutzbekleidungen, Förderbändern, Flockteppichen oder Schaumkunstleder. Besonders gut geeignet sind die erfindungsgemäßen Zubereitungen als haftvermittelnde Zusatzmittel bei der Beschichtung von Substraten mit gegenüber Isocyanatgruppen reaktionsfähigen Gruppen, insbesondere bei der Beschichtung von Garnen, Matten und Geweben aus Polyester- oder Polyamidfasern.

Anhand der nachfolgenden Beispiele wird die Erfindung näher erläutert, ohne dass dadurch eine Einschränkung der Erfindung bewirkt werden soll.

### Beispiele

Sofern nicht anders vermerkt, beziehen sich alle Teile und Prozentangaben auf das Gewicht.

Als Kenndaten der Produkte wurden der Festkörpergehalt (Dickschichtmethode: Deckel, 1 g Probe, 1 h 125 °C Konvektionsofen, Grundlage DIN EN ISO 3251), die Viskosität bei 23 °C (Rotationsviskosimeter VT550 der Fa. Haake GmbH, Karlsruhe) sowie der Gehalt an freiem TDI (Gaschromatographie, Hewlett Packard 5890 nach DIN ISO 55956) bestimmt. Die Bestimmung des Isocyanatgehaltes erfolgte nach EN ISO 11909.

### Ausgangsstoffe

Desmodur® T80: TDI-Isomerengemisch aus 80 Gew.-% 2,4-TDI und 20 Gew.-% 2,6-TDI, Bayer Material Science AG.

Vestinol® 9 DINP: Diisononylphthalat, Oxeno GmbH.

Vestinol® AH: Di-2-ethylhexylphthalat, Oxeno GmbH.

Adimoll® DO: Di-2-ethylhexyladipat, Lanxess Deutschland GmbH.

Benzoflex® 2088: Mischung aus Diethylenglycoldibenzoat, Triethylenglycoldibenzoat und Dipropylenglycoldibenzoat, Velsicol Chemical Corp.

Mesamoll®: Alkansulfonsäurephenylester, Lanxess Deutschland GmbH.

Mesamoll® II: Alkansulfonsäurephenylester mit ≤ 0,25 Gew.-% flüchtigen paraffinischen Verbindungen, Lanxess Deutschland GmbH.

Katalysator (Herstellung nach DE 24 52 532 A1): 188 Gewichtsteile Bisphenol A wurden mit 720 Teilen einer 25 %igen wässrigen Dimethylamin-Lösung und 425 Gewichtsteilen einer 40 %-igen wässrigen Formaldehyd-Lösung für zwei Stunden auf 80 °C erhitzt. Nach Erkalten wurde die organische Phase abgetrennt und bei 90 °C und 15 mbar eingedampft. Der Rückstand wurde in einer Mischung aus gleichen Volumenteilen Butylacetat und Xylol aufgelöst, so dass die erhaltene Katalysatorlösung 30 Gew.-% der gewünschten Mannich-Base enthielt. Die Mengenangaben in den folgenden Beispielen beziehen sich auf diese Katalysatorlösung.

### Vergleichsbeispiel 1 (nicht erfindungsgemäß)

180 Teile Desmodur® T80 wurden bei 45 °C in 489 Teilen Vestinol® 9 DINP mit 7,85 Teilen der Katalysatorlösung trimerisiert. Nach 84 Stunden wurde die Reaktion durch Zugabe von 4,65 Teilen para-Toluolsulfonsäuremethylester unterbrochen und bei 60 bis 70 °C eine Stunde nachgerührt. Der Feststoffgehalt wurde durch Zugabe von 13,4 Teilen Vestinol® 9 DINP auf 27 % eingestellt. Es wurde eine klare Lösung mit einem Isocyanatgehalt von 4,7 %, einer Viskosität bei 23 °C von 5.700 mPas und einem Gehalt an freiem TDI von 0,16 % erhalten.

### Vergleichsbeispiel 2 (nicht erfindungsgemäß)

180 Teile Desmodur® T80 wurden bei 45 °C in 414 Teilen Adimoll® DO mit 2,2 Teilen der Katalysatorlösung trimerisiert. Nach fünf Stunden wurde die Reaktion durch Zugabe eines Stoppers unterbrochen, da eine starke Trübung auftrat und somit kein homogenes Produkt erhalten wurde.

### Vergleichsbeispiel 3 (nicht erfindungsgemäß)

180 Teile Desmodur® T80 wurden bei 45 °C in 414 Teilen Benzoflex® 2088 mit 1,8 Teilen der Katalysatorlösung trimerisiert. Nach 84 Stunden wurde die Reaktion durch Zugabe von 1,65 Teilen para-Toluolsulfonsäuremethylester unterbrochen und bei 60 bis 70 °C für eine Stunde nachgerührt. Es wurde eine klare Lösung mit einem Isocyanatgehalt von 4,8 %, einer Viskosität bei 23 °C von > 200.000 mPas und einem Gehalt an freiem TDI von 1,09 % erhalten.

Vergleichsbeispiel 1 entspricht Beispiel 2 aus EP 1 711 546 A1 und dient dazu, die Eigenschaften der erfindungsgemäßen Haftvermittler-Zubereitungen mit dem Stand der Technik vergleichen zu können. Wie die nicht erfindungsgemäßen Vergleichsbeispiele 2 und 3 zeigen, hat die Auswahl des Lösemittels einen entscheidenden Einfluss auf das Ergebnis der Trimerisation. So kann die gewünschte Eigenschaftskombination durch Verwendung der im Stand der Technik beschriebenen phthalatfreien Weichmacher wie Di-2-ethylhexyladipat oder Alkylenglykoldibenzoat-Mischungen nicht erzielt werden.

### Beispiel 1

180 Teile Desmodur® T80 wurden bei 45 °C in 489 Teilen Mesamoll® mit 7,85 Teilen der Katalysatorlösung trimerisiert. Nach 84 Stunden wurde die Reaktion durch Zugabe von 4,65 Teilen para-Toluolsulfonsäuremethylester unterbrochen und bei 60 bis 70 °C eine Stunde nachgerührt. Der Feststoffgehalt beträgt ca. 27 %. Es wurde eine klare Lösung mit einem Isocyanatgehalt von 5,3 %, einer Viskosität bei 23 °C von 10.400 mPas und einem Gehalt an freiem TDI von 0,78 % erhalten.

### Beispiel 2

180 Teile Desmodur® T80 wurden bei 50 °C in 420 Teile Mesamoll® II mit 7,85 Teilen der Katalysatorlösung trimerisiert. Nach 84 Stunden wurde die Reaktion durch Zugabe von 4,65 Teilen para-Toluolsulfonsäuremethylester unterbrochen und bei 60 bis 70 °C eine Stunde nachgerührt. Der Feststoffgehalt wurde durch Zugabe von Mesamoll® II auf 26 % eingestellt. Es wurde eine klare Lösung mit einem Isocyanatgehalt von 4,8 %, einer Viskosität bei 23 °C von 11.600 mPas und einem Gehalt an freiem TDI von 0,25 % erhalten.

### Anwendungstechnische Prüfung und Prüfergebnisse

In einem praxisnahen Prüfsystem wurde Polyestergewebe mit einer PVC-Plastisol/Haftvermittlerbeschichtung versehen. Die Haftfestigkeit dieser Beschichtung wurde anschließend an einem normierten Teststreifen bestimmt. Dazu wurden mit einem Rakel Polyestergewebe mit einem haftvermittlerhaltigen Haftstrich und zwei haftvermittlerfreien Deckstrichen mit ansonsten gleicher Zusammensetzung versehen. Diese Beschichtungen wurden in einem Heizschrank ausgeliert und der weiteren Prüfung zugeführt. Bei der Prüfung der Haftfestigkeit wurden einige Zentimeter der Beschichtung von dem Gewebe abgelöst, um Beschichtung und Gewebe in die Zugmaschine einspannen zu können, die beide Schichten dann weiter auftrennte. Die ersten Zentimeter der Beschichtung sollen sich deshalb leicht von Hand trennen lassen. Dies wurde durch eine ca. 5 cm breite Antihaftimprägnierung (Tabelle 1) erreicht, die in dünner Schicht mit einem Handrakel auf einem Ende des Gewebes aufgetragen wurde.

**Tabelle 1: Zusammensetzung der Antihaftimprägnierung**

| **Bestandteil** | **Menge** |
|---|---|
| Cellit® 900, Bayer AG | 105 Teile |
| Ethylacetat | 595 Teile |
| Mesamoll® | 10 Teile |

Der Auftrag erfolgte einseitig auf der Seite des Gewebes, auf der auch der Haftstrich später aufgetragen wurde. Vor der Weiterverarbeitung wurde die Antihaft-Imprägnierung im Abzug getrocknet.

### Prüfeinrichtungen

Waage: Genauigkeit min.0,1 g
Rührer: hochtouriger Stabrührer
Umluftheizschränke: T = 140 °C bzw. 175 °C
Handrakel: 150 mm breit
Gummituchrakel: ca. 45 cm breit mit spitzem Rakel
Gummituchrakel: ca. 45 cm breit mit stumpfen Rakel
Polyester-Gewebe: Fa. Lückenhaus, 1.100 dtex, Bindung L 1/1, Einstellung 9/9 Fd/cm

Für die Prüfung wurden ca. 40 × 25 cm große Gewebeproben verwendet.

### Herstellung des PVC-Plastisols

**Tabelle 2: Zusammensetzung des PVC-Plastisols**

| **Bestandteile** | **Beschreibung** | **Menge** |
|---|---|---|
| Vestolit® B 7021 | Pasten-PVC, Vestolit GmbH | 30 Teile |
| Vestolit® E 8001 | Pasten-PVC, Vestolit GmbH | 30 Teile |
| Mesamoll®, Vestinol® AH | Weichmacher | 40 Teile |
| Omyalite® 95T | Calciumcarbonat, Omya Australia | 6 Teile |
| Naftovin® T90 | Stabilisator, Chemson GmbH | 2 Teile |
| Bayplast® Grün 8 GN | organisches Farbpigment, Lanxess Deutschland GmbH | 0,2 Teile |

Zur Herstellung des PVC-Plastisols wurden die in Tabelle 2 aufgeführten Ausgangsstoffe in einem Mischer der Firma Drais durch Rühren für 2,5 Stunden bei höchster Drehzahl, unter Wasserkühlung und im Vakuum vermischt.

### Haftstrich

Der Haftstrich basierend auf vorstehendem Plastisol mit variierenden Haftvermittlergehalten (siehe Tabelle 3) wurde mit einem Gummituch mit spitzem Rakel auf das Polyestergewebe aufgebracht. Das Auflagegewicht betrug dabei ca. 100 g/m2 und die Beschichtung erfolgte jeweils auf einer Fläche von ca. 30 × 20 cm. Anschließend wurden die Haftstriche durch zweiminütige Lagerung bei 140 °C im Umluftheizschrank vorgeliert, bevor die Deckstriche appliziert wurden.

### Erster Deckstrich

Der erste Deckstrich basierend auf vorstehendem Plastisol wurde mit einem Gummituch mit stumpfem Rakel (Auflagegewicht ca. 850 g/m2) aufgebracht und durch Temperung bei 140 °C im Heizschrank für 1 Minute vorgeliert.

### Beschichtung der Geweberückseite

Der anschließende Deckstrich auf der Rückseite der Gewebe verhinderte ein Einreißen und Ausfransen der Gewebe beim Trennen der Schichten durch die Zugmaschine. Die Beschichtung der Geweberückseite wurde mit einem Gummituch mit stumpfem Rakel (Auflagegewicht ca. 150 g/m2) aufgebracht und durch Temperung bei 140 °C im Heizschrank für 1 Minute vorgeliert.

### Zweiter Deckstrich

Der zweite Deckstrich ebenfalls basierend auf dem vorstehendend beschriebenen PVC-Plastisol wurde mit einem Gummituch mit stumpfem Rakel (Auflagegewicht ca. 1400g/m2) auf den ersten vorgelierten Deckstrich aufgebracht und durch Temperung bei 140 °C im Heizschrank für 2 Minuten vorgeliert.

Das Ausgelieren aller applizierten Schichten erfolgte anschließend durch zwölfminütige Lagerung bei 175 °C.

Aus den so hergestellten Gewebeproben wurden Prüfkörper in der Größe 5 × 26 cm ausgestanzt.

Anhand dieser Proben wurden dann die Haftfestigkeiten mittels einer Zugmaschine, Typ Lloyd M 5 K, bestimmt. Die erhaltenen Haftfestigkeitswerte geben die Kraft in Newton an, die notwendig ist, um 5 cm der Beschichtung vom Trägergewebe abzulösen (Schältest). Die in Tabelle 3 angegebenen Werte wurden durch Mittelung von mindestens drei Einzelmessungen erhalten.

**Tabelle 3: Prüfergebnisse im Schältest**

| **Haftvermittler-Zubereitung** | **Menge im Haftstrich** | **Haftfestigkeit** |
|---|---|---|
| Vergleichsbeispiel 1 (nicht erfindungsgemäß) | 2 % | 170 N/5 cm |
| Vergleichsbeispiel 1 (nicht erfindungsgemäß) | 4 % | 181 N/5 cm |
| Vergleichsbeispiel 1 (nicht erfindungsgemäß) | 6 % | 212 N/5 cm |
| Beispiel 1 | 2 % | 150 N/5 cm |
| Beispiel 1 | 4 % | 195 N/5 cm |
| Beispiel 1 | 6 % | 223 N/5 cm |
| Beispiel 2 | 2 % | 153 N/5 cm |
| Beispiel 2 | 4 % | 190 N/5 cm |
| Beispiel 2 | 6 % | 206 N/5 cm |

Wie die Messergebnisse zu den Beispielen 1 und 2 zeigen, liefert die Verwendung der erfindungsgemäßen phthalatfreien Haftvermitteler-Zubereitungen Haftfestigkeitswerte, wie sie auch mit der phthalathaltigen Haftvermitteler-Zubereitung aus dem Stand der Technik (Vergleichsbeispiel 1) erreicht werden. Die Haftvermittler aus den Vergleichsbeispielen 2 und 3 eigneten sich nicht zur Weiterverarbeitung, da diese entweder trübe (Vergleichsbeispiel 2) oder zu hochviskos waren (Vergleichsbeispiel 3), um homogene Beschichtungen erhalten zu können.

## Patentansprüche

1. Zubereitungen von isocyanatgruppenhaltigen Isocyanuraten mit einer Viskosität von < 30.000 mPas bei 23 °C und einem Anteil an freiem Diisocyanatotoluol von ≤ 1,0 Gew.-% (Summe aller Diisocyanatotoluol-Isomere), **dadurch gekennzeichnet, dass** sie
A) 15 bis 50 Gew.-% isocyanatgruppenhaltige Isocyanurate, die aus einer Mischung isomerer Diisocyanatotoluole enthaltend 65 bis 95 Gew.-% 2,4-Diisocyanatotoluol und 5 bis 35 Gew.-% 2,6-Diisocyanatotoluol unter Katalyse durch Dialkylaminogruppen enthaltende phenolische Katalysatoren und in Abwesenheit aliphatischer Hydroxyl- und/oder Urethangruppen hergestellt wurden, und
B) 85 bis 50 Gew.-% Alkansulfonsäurearylester-haltige, phthalatfreie Weichmacher enthalten.

2. Zubereitungen nach Anspruch 1, **dadurch gekennzeichnet, dass** sie 20 bis 35 Gew.-% isocyanatgruppenhaltige Isocyanurate und 80 bis 65 Gew.-% Alkansulfonsäurearylester-haltige, phthalatfreie Weichmacher enthalten.

3. Zubereitungen nach einem der Ansprüche 1 oder 2, **dadurch gekennzeichnet, dass** die isocyanatgruppenhaltigen Isocyanurate aus einer Mischung von 75 bis 85 Gew.-% 2,4-Diisocyanatotoluol und 15 bis 25 Gew.-% 2,6-Diisocyanatotoluol hergestellt werden.

4. Zubereitungen nach mindestens einem der Ansprüche 1 bis 3, **dadurch gekennzeichnet, dass** die Alkansulfonsäurearylester-haltigen, phthalatfreien Weichmacher > 90 Gew.-% Alkansulfonsäurearylester enthalten.

5. Zubereitungen nach mindestens einem der Ansprüche 1 bis 4, **dadurch gekennzeichnet, dass** es sich bei den Alkansulfonsäurearylestern um eine Mischung von Alkansulfonsäurephenylestern handelt.

6. Verwendung von Zubereitungen gemäß einem der Ansprüche 1 bis 5 als Haftvermittler für Beschichtungsmittel auf Basis von weichgemachtem Polyvinylchlorid.

7. Verwendung gemäß Anspruch 6, **dadurch gekennzeichnet dass** die Beschichtungsmittel zur Herstellung von Planen, Billboards, Traglufthallen und anderen textilen Bauten, flexiblen Behältern, Zeltdächern, Markisen, Schutzbekleidungen, Förderbändern, Flockteppichen oder Schaumkunstleder eingesetzt werden.

8. Beschichtungen für Textilien oder Gewebe erhältlich unter Verwendung von Zubereitungen gemäß einem der Ansprüche 1 bis 5.

9. Substrate beschichtet mit Beschichtungen gemäß Anspruch 8.

10. Substrate gemäß Anspruch 9, **dadurch gekennzeichnet, dass** es sich dabei um textile Polyester- oder Polyamidgewebe handelt.
